Europäisches Patentamt

European Patent Office

Office européen des brevets

(11) Publication number: **0 518 391 A1**

## EUROPEAN PATENT APPLICATION

(21) Application number: **92115541.2**

(22) Date of filing: **19.02.85**

(51) Int. Cl.⁵: **C07K 7/10, A61K 37/02**

(30) Priority: **23.02.84 US 583092**
     **14.05.84 US 610110**

(43) Date of publication of application:
   **16.12.92 Bulletin 92/51**

(60) Publication number of the earlier application in
   accordance with Art.76 EPC: **0 153 845**

(84) Designated Contracting States:
   **AT BE CH DE FR GB IT LI LU NL SE**

(71) Applicant: **THE SALK INSTITUTE FOR BIOLOGICAL STUDIES**
   **10010 North Torrey Pines Road**
   **La Jolla California 92037(US)**

(72) Inventor: **Rivier, Catherine Laure**
   **9674 Blackgold Road**
   **La Jolla, California 92037(US)**
   Inventor: **Rivier, Jean Edouard**
   **9674 Blackgold Road**
   **La Jolla, California 92037(US)**
   Inventor: **Vale, Jr., Wylie Walker**
   **1643 Valdez**
   **La Jolla, California 92037(US)**
   Inventor: **Brown, Marvin Ross**
   **484 Avenida Primavera**
   **Del Mar, California 92014(US)**

(74) Representative: **Allard, Susan Joyce et al**
   **BOULT, WADE & TENNANT, 27 Furnival Street**
   **London EC4A 1PO(GB)**

(54) CRF analogs.

㊐ Agonists of rCRF and oCRF are disclosed that can be administered to achieve a substantial elevation of ACTH, $\beta$-endorphin, $\beta$-lipotropin, other products of the pro-opiomelanocortin gene and corticosterone levels and/or a lowering of blood pressure over an extended period of time. One agonist which has been found to be particularly potent is:

```
              H-Ser-Gln-Glu-Pro-Pro-Ile-Ser-
   Leu-Asp-Leu-Thr-Phe-His-Leu-Leu-Arg-Glu-Met-Leu-Glu-Met-
   Ala-Lys-Ala-Glu-Gln-Glu-Ala-Glu-Gln-Ala-Ala-Leu-Asn-Arg-
   Leu-Leu-Leu-Glu-Glu-Ala-NH₂.
```

A number of substitutions may also be made throughout the chain. Similar peptides which function as CRF antagonists are created by deleting the first 7, 8 or 9 N-terminal residues. These analogs or pharmaceutically or veterinarily acceptable salts thereof, dispersed in a pharmaceutically or veterinarily acceptable liquid or solid carrier, can be administered to mammals, including humans.

This invention is directed to peptides and to methods for pharmaceutical treatment of mammals using such peptides. More specifically, the invention relates to the hentetracontapeptide CRF, to analogs of CRF, to pharmaceutical compositions containing CRF or such analogs and to methods of treatment of mammals using CRF or such analogs.

The hypothalamus plays a key role in the regulation of adenohypophysial corticotropic cells secretory functions. Factors in hypothalamus increase the rate of ACTH secretion by the pituitary gland. A physiologic corticotropin releasing factor (CRF), i.e., ovine CRF (oCRF), was characterized in 1981 and disclosed in U.S. Patent No. 4,415,558 to have the formula:

$$H-Ser-Gln-Glu-Pro-Pro-Ile-Ser-Leu-Asp-Leu-Thr-Phe-His-Leu-$$
$$Leu-Arg-Glu-Val-Leu-Glu-Met-Thr-Lys-Ala-Asp-Gln-Leu-Ala-$$
$$Gln-Gln-Ala-His-Ser-Asn-Arg-Lys-Leu-Leu-Asp-Ile-Ala-NH_2.$$

Rat CRF(rCRF) has been characterized as having the formula:

$$H-Ser-Glu-Glu-Pro-Pro-Ile-Ser-Leu-Asp-Leu-$$
$$Thr-Phe-His-Leu-Leu-Arg-Glu-Val-Leu-Glu-Met-Ala-Arg-Ala-$$
$$Glu-Gln-Leu-Ala-Gln-Gln-Ala-His-Ser-Asn-Arg-Lys-Leu-Met-$$
$$Glu-Ile-Ile-NH_2$$

and may alternatively be referred to as rat Amunine. The formula of human CRF has apparently been determined to be the same as that of rCRF. Synthetic rCRF and oCRF stimulate ACTH and $\beta$-endorphin activites in vitro and in vivo and substantially lower blood pressure for an extended time period.

Analogs of these 41-residue CRF peptides, having the following formula which are competitive antagonists of the natural peptides:

$$Y-Q-leu-R_{11}-R_{12}-R_{13}-leu-leu-$$

$$Arg-R_{17}-R_{18}-R_{19}-Glu-R_{21}-R_{22}-R_{23}-R_{24}-R_{25}-$$
$$R_{26}-R_{27}-R_{28}-R_{29}-Gln-ala-R_{32}-R_{33}-Asn-Arg-$$
$$R_{36}-R_{37}-R_{38}-R_{39}-R_{40}-R_{41}-NH_2$$

wherein Y is an acyl group having 7 or less carbon atoms or hydrogen; Q is $R_8$-$R_9$ or $R_8$ or $R_9$ or desQ; $R_1$ is Ser, D-Ser or des $R_1$, $R_2$ is Gln, pGlu, Glu, D-pGlu or des $R_2$; $R_3$ is Glu, Gly, D-Tyr or des $R_3$; $R_4$ is Pro, D-Pro or des $R_4$; $R_5$ is Pro or des$R_5$; $R_8$, $R_{12}$, $R_{19}$ and $R_{24}$ are selected from the group consisting of leu, Ile, ala, Gly, Val, Nle, Phe and Gln; $R_9$ is Asp or Glu; $R_{11}$ is Thr or Ser; $R_{13}$ is His, Tyr or Glu; $R_{17}$ is Glu or Lys; $R_{18}$ is Val, Nle or Met; $R_{21}$ is Met, Nva, Ile, ala, leu, Nle, Val, Phe or Gln; $R_{22}$ is ala, Thr, Asp or Glu; $R_{23}$ is Arg, Orn, Har or Lys; $R_{25}$ is Asp or Glu; $R_{26}$ is Gln, Asn or Lys; $R_{27}$ is leu, Ile, ala, Val, Nva, Met, Nle, Phe, Asp, Asn, Gln or Glu; $R_{28}$ is ala, Arg or Lys; $R_{29}$ is Gln or Glu, $R_{32}$ is His, Gly, Tyr or ala; $R_{33}$ is Ser, Asn, leu, Thr or ala; $R_{36}$ is Lys, Orn, Arg, Har or Leu; $R_{37}$ is leu or Tyr; $R_{38}$ is Met or leu; $R_{39}$ is Glu or Asp; $R_{40}$ is Ile, Thr, Glu, ala, Val, leu, Nle, Phe, Nva, Gly or Gln; $R_{41}$ is ala, Ile, Gly, Val, Leu, Nle, Phe, Gln or des $R_{41}$; or a nontoxic addition salt thereof.

Pharmaceutical compositions in accordance with the invention include such CRF analogs, or nontoxic addition salts thereof, dispersed in a pharmaceutically or veterinarily acceptable liquid or solid carrier. The administration of such peptides or pharmaceutically or veterinarily acceptable addition salts thereof to mammals, particularly humans, in accordance with the invention may be carried out for the regulation of secretion of ACTH, $\beta$-endorphin, $\beta$-lipotropin, other products of the pro-opiomelanocortin gene and corticosterone and/or for the lowering of blood pressure and/or for affecting mood, behavioral and gastro-intestinal functions and autonomic nervous system activities.

The nomenclature used to define the peptides is that specified by Schroder & Lubke, "The Peptides", Academic Press (1965) wherein, in accordance with conventional representation, the amino group appears to the left and the carboxyl group to the right. The standard 3-letter abbreviations to identify the alpha-amino acid residues, and where the amino acid residue has isomeric forms, it is the L-form of the amino acid that is represented unless otherwise expressly indicated, e.g. Ser = L-serine, Nle = L-norleucine, Nva = norvaline, Har = homoarginine, Orn = ornithine etc. In addition the following abbreviations are used: leu = either L-leucine or $C^{\alpha}CH_3$-L-leucine (CML) and ala = either L-alanine or $C^{\alpha}CH_3$-L-alanine(CMA).

The invention provides analogs of CRF having the following Formula (I):

$$Y-Q-leu-R_{11}-R_{12}-R_{13}-$$
$$leu-leu-Arg-R_{17}-R_{18}-R_{19}-Glu-R_{21}-R_{22}-R_{23}-$$
$$R_{24}-R_{25}-R_{26}-R_{27}-R_{28}-R_{29}-Gln-ala-R_{32}-R_{33}-$$
$$Asn-Arg-R_{36}-R_{37}-R_{38}-R_{39}-R_{40}-R_{41}-NH_2$$

wherein Y is an acyl group having 7 or less carbon atoms or hydrogen; Q is $R_8$-$R_9$ or $R_8$ or $R_9$ or desQ; $R_1$ is Ser, D-Ser or des $R_1$; $R_2$ is Gln, pGlu, Glu, D-pGlu or des $R_2$; $R_3$ is Glu, Gly, D-Tyr or des $R_3$; $R_4$ is Pro, D-Pro or des $R_4$; $R_5$ is Pro or des$R_5$; $R_8$, $R_{12}$, $R_{19}$ and $R_{24}$ are selected from the group consisting of leu, Ile, ala, Gly, Val, Nle, Phe and Gln; $R_9$ is Asp or Glu; $R_{11}$ is Thr or Ser; $R_{13}$ is His, Tyr or Glu; $R_{17}$ is Glu or Lys; $R_{18}$ is Val, Nle or Met; $R_{21}$ is Met, Nva, Ile, ala, leu, Nle, Val, Phe or Gln; $R_{22}$ is ala, Thr, Asp or Glu; $R_{23}$ is Arg, Orn, Har or Lys; $R_{25}$ is Asp or Glu; $R_{26}$ is Gln, Asn or Lys; $R_{27}$ is leu, Ile, ala, Val, Nva, Met, Nle, Phe, Asp, Asn, Gln or Glu; $R_{28}$ is ala, Arg or Lys; $R_{29}$ is Gln or Glu, $R_{32}$ is His, Gly, Tyr or ala; $R_{33}$ is Ser, Asn, leu, Thr or ala; $R_{36}$ is Lys, Orn, Arg, Har or Leu; $R_{37}$ is leu or Tyr; $R_{38}$ is Met or leu; $R_{39}$ is Glu or Asp; $R_{40}$ is Ile, Thr, Glu, ala, Val, leu, Nle, Phe, Nva, Gly or Gln; $R_{41}$ is ala, Ile, Gly, Val, Leu, Nle, Phe, Gln or des $R_{41}$.

CRF agonists have been synthesized which are at least as potent as and often more potent than native CRF. These analogs preferably include the following residues having a high alpha-helical forming potential: $R_1$ is Ser, $R_2$ is Gln or Glu, $R_3$ is Glu, $R_4$ and $R_5$ are Pro, $R_8$ is leu, $R_{11}$ is Thr, $R_{12}$ is Phe or leu, $R_{13}$ is His or Glu, $R_{17}$ is Glu, $R_{18}$ and $R_{21}$ are Met or Nle, $R_{19}$ and $R_{37}$ are leu, $R_{22}$ and $R_{41}$ are ala, $R_{23}$ is Lys, $R_{24}$ and $R_{28}$ are ala, $R_{25}$ and $R_{39}$ are Glu, $R_{26}$ is Gln, $R_{27}$ is Glu or leu, $R_{29}$ is Glu, $R_{32}$ is His or ala, $R_{33}$ is Ser or leu, $R_{38}$ is Leu and $R_{40}$ is Ile or Glu. One analog which has been found to be particularly potent is:

$$H-Ser-Gln-Glu-Pro-Pro-Ile-Ser-Leu-Asp-Leu-Thr-Phe-His-$$
$$Leu-Leu-Arg-Glu-Met-Leu-Glu-Met-Ala-Lys-Ala-Glu-Gln-Glu-$$
$$Ala-Glu-Gln-Ala-Ala-Leu-Asn-Arg-Leu-Leu-Leu-Glu-Glu-$$
$$Ala-NH_2$$

and is hereinafter referred to as AHC (for alpha-helical CRF); it remains potent even if shortened at the N-terminus by from one to five residues. However, if shortened to eliminate residues 1 to 7, 1 to 8 or 1 to 9, competitive antagonists of CRF are formed.

The peptides are synthesized by a suitable method, such as by exclusively solid-phase techniques, by partial solid-phase techniques, by fragment condensation or by classical solution addition. Certain CRF analogs which do not include D-isomer residues or unnatural amino acid residues may also be synthesized by recently developed recombinant DNA techniques.

Common to chemical syntheses of peptides is the protection of the labile side chain groups of the various amino acid moieties with suitable protecting groups which will prevent a chemical reaction from occurring at that site until the group is ultimately removed. Usually also common is the protection of an alpha-amino group on an amino acid or a fragment while that entity reacts at the carboxyl group, followed by the selective removal of the alpha-amino protecting group to allow subsequent reaction to take place at that location. Accordingly, it is common that, as a step in the synthesis, an intermediate compound is produced which includes each of the amino acid residues located in its desired sequence in the peptide chain with various of these residues having side-chain protecting groups.

Also considered to be within the scope of the present invention are intermediates of the Formula (II):

3

$$X^1 - R_8 - R_9(X^5) - leu - R_{11}(X^2) - R_{12}(X^4) - R_{13}(X \text{ or } X^5) - leu - leu -$$

$$Arg(X^3) - R_{17}(X^5 \text{ or } X^6) - R_{18} - R_{19}(X^4) - Glu(X^5) - R_{21} - R_{22}(X^2 \text{ or } X^5) - R_{23}(X^3 \text{ or } X^6) - R_{24}(X^4) - R_{25}(X^5) - R_{26}(X^4 \text{ or } X^6) - R_{27}(X^4 \text{ or } X^5) - R_{28}(X^3 \text{ or } X^6) - R_{29}(X^4 \text{ or } X^5) - Gln(X^4) - Ala - R_{32}(X) - R_{33}(X^2 \text{ or } X^4) - Asn(X^4) - Arg(X^3) - R_{36}(X^6) - R_{37}(X) - R_{38} - R_{39}(X^5) - R_{40}(X^2 \text{ or } X^4 \text{ or } X^5) - R_{41}(X^4) - X^7,$$

or versions thereof which are shortened at the N-terminus as set forth hereinbefore, wherein: the R-groups are as hereinbefore defined.

$X^1$ is either hydrogen or an $\alpha$-amino protecting group. The $\alpha$-amino protecting groups contemplated by $X^1$ are those known to be useful in the art in the step-wise synthesis of polypeptides. Among the classes of $\alpha$-amino protecting groups covered by $X^1$ are (1) acyl-type protecting groups, such as formyl, acrylyl(Acr), benzoyl(Bz) and acetyl(Ac) which are preferably used only at the N-terminal; (2) aromatic urethan-type protecting groups, such as benzyloxycarbonyl(Z) and substituted Z, such as p-chlorobenzyloxycarbonyl, p-nitrobenzyloxycarbonyl, p-bromobenzyloxycarbonyl, p-methoxybenzyloxycarbonyl; (3) aliphatic urethan protecting groups, such as t-butyloxycarbonyl (BOC), diisopropylmethoxycarbonyl, isopropyloxycarbonyl, ethoxycarbonyl, allyloxycarbonyl; (4) cycloalkyl urethan-type protecting groups, such as fluorenylmethyloxycarbonyl(FMOC), cyclopentyloxycarbonyl, adamantyloxycarbonyl,and cyclohexyloxycarbonyl; and (5) thiourethan-type protecting groups, such as phenylthiocarbonyl. The preferred $\alpha$-amino protecting group is BOC.

$X^2$ is a protecting group for the hydroxyl group of Thr and Ser and is preferably selected from the class consisting of acetyl(Ac) , benzoyl(Bz), tert-butyl, triphenylmethyl(trityl), tetrahydropyranyl, benzyl ether(Bzl) and 2,6-dichlorobenzyl (DCB). The most preferred protecting group is Bzl. $X^2$ can be hydrogen, which means there is no protecting group on the hydroxyl group.

$X^3$ is a protecting group for the guanidino group of Arg or Har preferably selected from the class consisting of nitro, p-toluenesulfonyl(Tos), Z, adamantyloxycarbonyl and BOC, or is hydrogen. Tos is most preferred.

$X^4$ is hydrogen or a protecting group, preferably xanthyl(Xan), for the amido group of Asn or Gln.

$X^5$ is hydrogen or an ester-forming protecting group for the $\beta$- or $\gamma$-carboxyl group of Asp or Glu, preferably selected from the class consisting of benzyl, 2,6-dichlorobenzyl, methyl, ethyl and t-butyl ester. OBzl is most preferred.

$X^5$ is hydrogen or a protecting group for the side chain amino substituent of Lys or Orn. Illustrative of suitable side chain amino protecting groups are Z, 2-chlorobenzyloxycarbonyl(2-Cl-Z), Tos, t-amyloxycarbonyl(Aoc), BOC and aromatic or aliphatic urethan-type protecting groups as specified hereinbefore.

When His is present, X is hydrogen or a protecting group for the imidazole nitrogen such as Tos or 2,4-dinitrophenyl(DNP), and when Tyr is present, X is hydrogen or a protecting group for the hydroxyl group such as DCB. When Met is present, the sulfur may be protected, if desired, with oxygen.

The selection of a side chain amino protecting group is not critical except that it should must be one which is not removed during deprotection of the $\alpha$-amino groups during the synthesis. Hence, the $\alpha$-amino protecting group and the side chain amino protecting group cannot be the same.

$X^7$ is $NH_2$, a protecting group such as an ester or an anchoring bond used in solid phase synthesis for linking to a solid resin support, preferably one represented by the formulae:

-NH-benzhydrylamine (BHA) resin support and -NH-paramethylbenzhydrylamine (MBHA) resin support. Cleavage from a BHA or MBHA resin directly gives the CRF analog amide. By employing a methyl-derivative of such a resin, a methyl-substituted amide can be created.

In the formula for the intermediate, at least one of X, $X^1$, $X^2$, $X^3$, $X^4$, $X^5$ and $X^5$ is a protecting group. The particular amino acid chosen for each the R-group determines whether there will also be a protecting group attached as specified hereinbefore and as generally known in the art. In selecting a particular side chain protecting group to be used in the synthesis of the peptides, the following rules are followed: (a) the protecting group should be stable to the reagent and under the reaction conditions selected for removing the $\alpha$-amino protecting group at each step of the synthesis, (b) the protecting group should retain its protecting properties and not be split off under coupling conditions and (c) the side chain protecting group must be removable, upon the completion of the synthesis containing the desired amino acid sequence, under reaction conditions that will not alter the peptide chain.

Thus, the present invention is also considered to provide a process for the manufacture of compounds defined by the Formula (I) comprising (a) forming a peptide having at least one protecting group and having the Formula (II) wherein: X, $X^1$, $X^2$, $X^3$, $X^4$, $X^5$ and $X^5$ are each either hydrogen or a protecting group, and $X^7$ is either a protecting group or an anchoring bond to resin support or $NH_2$ and (b) splitting off the protective group or groups or anchoring bond from said peptide of the Formula (II) and (c) if desired, converting a resulting peptide into a nontoxic addition salt thereof.

When the peptides are prepared by chemical synthesis, they are preferably prepared using solid phase synthesis, such as that described by Merrifield, J. Am. Chem. Soc., 85, p 2149 (1964), although other equivalent chemical syntheses known in the art can also be used as previously mentioned. Solid-phase synthesis is commenced from the C-terminal end of the peptide by coupling a protected α-amino acid to a suitable resin as generally set forth in U.S. Patent No. 4,244,946 issued Jan. 21, 1981 to Rivier et al., the disclosure of which is incorporated herein by reference. Such a starting material for rCRF analogs can be prepared by attaching α-amino-protected Ile to a BHA resin.

Ile protected by BOC is coupled to the BHA resin using methylene chloride and dimethylformamide (DMF). Following the coupling of BOC-Ile to the resin support, the α-amino protecting group is removed, as by using trifluoroacetic acid(TFA) in methylene chloride, TFA alone or with HCl in dioxane. Preferably 50 volume % TFA in methylene chloride is used with 0-5 weight % 1,2 ethanedithiol. The deprotection is carried out at a temperature between about 0°C and room temperature. Other standard cleaving reagents and conditions for removal of specific α-amino protecting groups may be used as described in Schroder & Lubke, "The Peptides", 1 pp 72-75 (Academic Press 1965).

After removal of the α-amino protecting group of Ile, the remaining α-amino- and side chain-protected amino acids are coupled step-wise in the desired order to obtain the intermediate compound defined hereinbefore. As an alternative to adding each amino acid separately in the synthesis, some of them may be coupled to one another prior to addition to the solid phase reactor. The selection of an appropriate coupling reagent is within the skill of the art. Particularly suitable as coupling reagents are N,N'-dicyclohexyl carbodiimide(DCCI) and N,N'-diisopropyl carbodiimide(DICI).

The activating reagents used in the solid phase synthesis of the peptides are well known in the peptide art. Examples of suitable activating reagents are carbodiimides, such as N,N'-diisopropyl carbodiimide and N-ethyl-N'-(3-dimethylaminopropyl)carbodiimide. Other activating reagents and their use in peptide coupling are described by Schroder & Lubke, supra, in Chapter III and by Kapoor, J. Phar. Sci., 59, pp 1-27 (1970).

Each protected amino acid or amino acid sequence is introduced into the solid phase reactor in about a fourfold excess, and the coupling is carried out in a medium of dimethylformamide(DMF):$CH_2Cl_2$ (1:1) or in DMF or $CH_2Cl_2$ alone. In instances where the coupling is carried out manually, the success of the coupling reaction at each stage of the synthesis is monitored by the ninhydrin reaction, as described by E. Kaiser et al., Anal. Biochem. 34, 595 (1970). In cases where incomplete coupling occurs, the coupling procedure is repeated before removal of the α-amino protecting group prior to the coupling of the next amino acid. The coupling reactions can be performed automatically, as on a Beckman 990 automatic synthesizer, using a program such as that reported in Rivier et al., Biopolymers, 1978, 17, pp.1927-1938.

After the desired amino acid sequence has been completed, the intermediate peptide is removed from the resin support by treatment with a reagent, such as liquid hydrogen fluoride, which not only cleaves the peptide from the resin but also cleaves all remaining side chain protecting groups $X^2$, $X^3$, $X^4$, $X^5$ and $X^5$ and the α-amino protecting group $X^1$ (unless it is an acyl group which is intended to be present in the final peptide), to obtain the peptide. When using hydrogen fluoride for cleaving, anisole or cresole and methylethyl sulfide are included in the reaction vessel as scavengers. When Met is present in the sequence, the BOC protecting group may be cleaved with trifluoroacetic acid(TFA)/ethanedithiol prior to cleaving the peptide from the resin to eliminate S-alkylation.

The following Example, which is not an Example of the invention, is included for reference purposes and sets for the preferred method for synthesizing CRF analogs by the solid-phase technique.

EXAMPLE I

The synthesis of [Glu$^{29}$)-rCRF having the formula:

H-Ser-Glu-Glu-Pro-Pro-Ile-Ser-Leu-Asp-Leu-Thr-
Phe-His-Leu-Leu-Arg-Glu-Val-Leu-Glu-Met-Ala-Arg-Ala-Glu-
Gln-Leu-Ala-Glu-Gln-Ala-His-Ser-Asn-Arg-Lys-Leu-Met-Glu-
Ile-Ile-NH$_2$

is conducted in a stepwise manner on a MBHA hydrochloride resin, such as available from Bachem, Inc., having a substitution range of about 0.1 to 0.5 mmoles/gm. resin.

The synthesis is performed on an automatic Beckman 990B peptide synthesizer using a suitable program, preferably as follows:

| STEP | REAGENTS AND OPERATIONS | MIX TIMES MIN. |
|---|---|---|
| 1 | $CH_2Cl_2$ wash-80 ml. (2 times) | 3 |
| 2 | Methanol (MeOH) wash-30 ml. (2 times) | 3 |
| 3 | $CH_2Cl_2$ wash-80 ml. (3 times) | 3 |
| 4 | 50 percent TFA plus 5 percent 1,2-ethanedithiol in $CH_2Cl_2$-70 ml. (2 times) | 12 |
| 5 | Isopropanol wash-80 ml. (2 times) | 3 |
| 6 | TEA 12.5 percent in $CH_2Cl_2$-70 ml. (2 times) | 5 |
| 7 | MeOH wash-40 ml. (2 times) | 2 |
| 8 | $CH_2Cl_2$ wash-80 ml. (3 times) | 3 |
| 9 | Boc-amino acid (10 mmoles) in 30 ml. of either DMF or $CH_2Cl_2$, depending upon the solubility of the particular protected amino acid, (1 time) plus DCCI (10 mmoles) in $CH_2Cl_2$ | 30-300 |

Coupling of BOC-Ile results in the substitution of about 0.35 mmol. Ile per gram of resin. All solvents that are used are carefully degassed, preferably by sparging with an inert gas, e.g. helium or nitrogen, to insure the absence of oxygen that might undesirably oxidize the sulfur of the Met residue.

After deprotection and neutralization, the peptide chain is built step-by-step on the resin. Generally, one to two mmol. of BOC-protected amino acid in methylene chloride is used per gram of resin, plus one equivalent of 2 molar DCCI in methylene chloride, for two hours. When BOC-Arg(Tos) is being coupled, a mixture of 50% DMF and methylene chloride is used. Bzl is used as the hydroxyl side-chain protecting group for Ser and Thr. P-nitrophenyl ester(ONp) is used to activate the carboxyl end of Asn or Gln, and for example, BOC-Asn(ONp) is coupled overnight using one equivalent of HOBt in a 50% mixture of DMF and methylene chloride. The amido group of Asn or Gln is protected by Xan when DCCI coupling is used instead of the active ester method. 2-Cl-Z is used as the protecting group for the Lys side chain. Tos is used to protect the guanidino group of Arg and the imidazole group of His, and the side chain carboxyl group of Glu or Asp is protected by OBzl. At the end of the synthesis, the following composition is obtained

BOC-Ser(Bzl)-Glu(OBzl)-
Glu(OBzl)-Pro-Pro-Ile-Ser(Bzl)-Leu-Asp(OBzl)-Leu-Thr(Bzl)-
Phe-His(Tos)-Leu-Leu-Arg(Tos)-Glu(OBzl)-Val-Leu-Glu(OBzl)-
Met-Ala-Arg(Tos)-Ala-Glu(OBzl)-Gln(Xan)-Leu-Ala-Glu(OBzl)-
Gln(Xan)-Ala-His(Tos)-Ser(Bzl)-Asn(Xan)-Arg(Tos)-Lys
(2-Cl-Z)-Leu-Met-Glu(OBzl)-Ile-Ile-resin support.

Xan may have been partially or totally removed by TFA treatment used to deblock the α-amino protecting group.

In order to cleave and deprotect the resulting protected peptide-resin, it is treated with 1.5 ml. anisole, 0.5 ml. of methylethylsulfide and 15 ml. hydrogen fluoride (HF) per gram of peptide-resin, first at -20°C. for 20 min. and then at 0.°C. for one-half hour. After elimination of the HF under high vacuum, the resin-peptide is washed alternately with dry diethyl ether and chloroform, and the peptides are then extracted with de-gassed 2N aqueous acetic acid and separated from the resin by filtration.

The peptide is purified by gel permeation followed by semi-preparative HPLC as described in Rivier et al., Peptides: Structure and Biological Function (1979) pp. 125-128, and Rivier et al., J. Chromatography - (1983). The chromatographic fractions are carefully monitored by HPLC, and only the fractions showing substantial purity were pooled.

To check whether the precise sequence was achieved, the rCRF analog was hydrolyzed in sealed evacuated tubes containing constant boiling HCl, 3$\mu$l of thioglycol/ml. and 1 nmol of Nle (as an internal standard) for 9 hours at 140°C. Amino acid analyses of the hydrolysates using a Beckman 121 MB amino acid analyzer showed the following amino acid ratios: Asx(1.9), Thr(0.8), Ser(3.1), Glx(9.0), Pro(2.1), Ala(3.8), Val(0.9), Met(1.9), Ile(2.6), Leu(7.0), Phe(0.9), Lys(1.0), His(2.0) and Arg(3.0), which confirmed that the 41-residue peptide structure had been obtained.

## EXAMPLE II

The synthetic peptide AHC(9-41) having the formula:

$$\text{H-Asp-Leu-Thr-Phe-His-Leu-Leu-Arg-Glu-Met-Leu-}$$
$$\text{Glu-Met-Ala-Lys-Ala-Glu-Gln-Glu-Ala-Glu-Gln-Ala-Ala-Leu-}$$
$$\text{Asn-Arg-Leu-Leu-Leu-Glu-Glu-Ala-NH}_2$$

is synthesized generally in accordance with the procedure set forth in Example I.

## EXAMPLE III

The synthetic CRF antagonist from Example II is examined for its effects on the secretion of ACTH and $\beta$-endorphin in vitro and the synthetic AHC peptide is also examined in vivo. The effectiveness of the synthetic CRF antagonist to block the secretion of ACTH and $\beta$-endorphin by cultured rat pituitary cells is measured using the procedure as generally set forth in Vale et al., Endocrinology, 91, 562 (1972). In vivo testing is carried out using the general procedure set forth in C. Rivier et al., Science, 218, 377 (1982).

Based upon five independent experiments what we herein term the standard antagonist, AHC (9-41), blocks the secretion of ACTH due to 1 nMoCRF by 50%, at a concentration of 197 ± 72 nM. The specificity of this inhibition is demonstrated by the finding of no effect of the standard antagonist on the GRF-stimulated secretion of GH, the GnRH-stimulated secretion of LH and FSH or the TRF-stimulated secretion of TSH and prolactin. The effects of the antagonist on a number of different concentrations of oCRF and the ability of several different concentrations of AHC (9-41) to inhibit ACTH secretion stimulated by a constant dose of oCRF (1 nM) are considered to demonstrate competitive inhibition.

The in vivo effect of the CRF antagonist is tested on the spontaneous ACTH release by adrenalectomized rats. The iv injection of 3 mg/kg BW (2.7 nmole) causes a marked decrease in plasma ACTH levels (measured as described in Vale et al. Science, 213, 1394, 1981), which is statistically significant for 2 hours. In the intact, non-anesthetized rats, the antagonist induces a dose-related inhibition of CRF-induced ACTH secretion, which is significant at the 0.09 $\mu$mole dose level. The antagonist AHC (9-41) also prevents most, but not all, of the ACTH rise due to ether-exposure.

These results indicate that administration of the CRF antagonist reduces the spontaneous ACTH release observed after removal of the corticosteroid feedback, totally blocks the ACTH secretion caused by CRF, and inhibits most of the stressor-induced ACTH release in intact rats. Such data are comparable to those previously obtained in our laboratory with an antiserum to CRF which demonstrate the role played by endogenous CRF in regulating ACTH secretion, Rivier, C. et al., Science, 218, 377-9(1982).

These results confirm the hypothesis that CRF is indeed a critical element in the regulation of ACTH under several circumstances. In addition, that CRF antagonists can partially block the ether-exposure-induced activation of the sympathetic nervous system suggest a much broader role for this neuropeptide in mediating the response to stressful stimuli.

Synthetic hCRF has been shown to be a powerful stimulator of ACTH and $\beta$-END-LI secretion in vivo in several rat preparations. Plasma levels of ACTH and $\beta$-END-LI are elevated for at least 5-20 minutes following the intraveneous administration of hCRF to nembutal-anesthesized male rats and to quiescent male or female rats with indwelling intravenous cannulae. In addition, hCRF is found to have a dramatic effect to lower blood pressure in rats and dogs.

EXAMPLE: IV

The peptide [CML[10,15,27,37], CMA[22,32,41]]-AHC(9-41) having the formula:

```
                                    H-Asp-CML-Thr-Leu-
Glu-CML-CML-Arg-Glu-Met-CML-Glu-Met-CMA-Lys-Ala-Glu-Gln-
CML-Ala-Glu-Gln-Ala-CMA-CML-Asn-Arg-Leu-CML-Leu-Glu-Glu-.
CMA-NH_2.
```

Testing in accordance with the general procedure set forth in Example III shows that it likewise inhibits the secretion of ACTH and $\beta$-END-LI.

EXAMPLE V

The peptide [Nle[18,21]]-AHC(9-41) having the formula:

```
        H-Asp-Leu-Thr-Phe-His-Leu-Leu-Arg-Glu-Nle-Leu-
Glu-Nle-Ala-Lys-Ala-Glu-Gln-Glu-Ala-Glu-Gln-Ala-Ala-Leu-
Asn-Arg-Leu-Leu-Leu-Glu-Glu-Ala-NH_2
```

is synthesized. Testing in accordance with the general procedure set forth in Example III shows that it likewise inhibits the secretion of ACTH and $\beta$-END-LI.

EXAMPLE VI

The peptide [Nle[18,21]]-AHC(8-41) having the formula:

```
      H-Leu-Asp-Leu-Thr-Phe-His-Leu-Leu-Arg-Glu-Nle-
Leu-Glu-Nle-Ala-Lys-Ala-Glu-Gln-Glu-Ala-Glu-Gln-Ala-Ala-
Leu-Asn-Arg-Leu-Leu-Leu-Glu-Glu-Ala-NH_2
```

is synthesized. Testing in accordance with the general procedure set forth in Example III shows that it likewise inhibits the secretion of ACTH and $\beta$-END-LI.

EXAMPLE VII

The peptide [Glu[13,22], Leu[12], Lys[26]]-AHC(8-41) having the formula:

```
                    H-Leu-Asp-Leu-Thr-Leu-Glu-Leu-Leu-
Arg-Glu-Met-Leu-Glu-Met-Glu-Lys-Ala-Glu-Lys-Glu-Ala-Glu-
Gln-Ala-Ala-Leu-Asn-Arg-Leu-Leu-Leu-Glu-Glu-Ala-NH_2.
```

Testing in accordance with the general procedure set forth in Example XLIII shows that it likewise inhibits the secretion of ACTH and $\beta$-END-LI.

8

EXAMPLE VIII

The peptide [Leu[12], Glu[13]]-AHC(9-41) having the formula:

```
        H-Asp-Leu-Thr-Leu-Glu-Leu-Leu-Arg-
Glu-Met-Leu-Glu-Met-Ala-Lys-Ala-Glu-Gln-Glu-Ala-Glu-Gln-
Ala-Ala-Leu-Asn-Arg-Leu-Leu-Leu-Glu-Glu-Ala-NH₂.
```

Testing in accordance with the general procedure set forth in Example III shows that it likewise inhibits the secretion of ACTH and $\beta$-END-LI.

EXAMPLE IX

The peptide [CML[10,14,19,27,33,38]]-AHC(9-41) having the formula:

```
        H-Asp-CML-Thr-Leu-Glu-CML-Leu-Arg-
Glu-Met-CML-Glu-Met-Ala-Lys-Ala-Glu-Gln-CML-Ala-Glu-Gln-
Ala-Ala-CML-Asn-Arg-Leu-CML-Leu-Glu-Glu-Ala-NH₂.
```

Testing in accordance with the general procedure set forth in Example III shows that it likewise stimulates the secretion of ACTH and $\beta$-END-LI and causes a very significant lowering of blood pressure.

EXAMPLE X

The peptide [Nle[18,21]]-AHC(10-41) having the formula:

```
        H-Leu-Thr-Phe-His-Leu-Leu-Arg-Glu-Nle-Leu-
Glu-Nle-Ala-Lys-Ala-Glu-Gln-Glu-Ala-Glu-Gln-Ala-Ala-Leu-
Asn-Arg-Leu-Leu-Leu-Glu-Glu-Ala-NH₂
```

is synthesized. Testing in accordance with the general procedure set forth in Example II shows that it likewise inhibits the secretion of ACTH and $\beta$-END-LI.

CRF profoundly stimulates the pituitary-adrenalcortical axis, and CRF analogs should be useful to stimulate the functions of this axis in some types of patients with low endogenous glucocorticoid production. For example, CRF should be useful in restoring pituitary-adrenal function in patients having received exogenous glucocorticoid therapy whose pituitary-adrenalcortical functions remain supressed. For example, CRF antagonists may be useful in regulating pituitary-adrenal function in patients having pituitary Cushings disease or any CRF-sensitive tumor.

Most other regulatory peptides have been found to have effects upon the central nervous system and upon the gastrointestinal tract. Because ACTH and $\beta$-END secretion is the "sine qua non" of mammal's response to stress, it was not surprising that CRF has significant effects on the brain as a mediator of the body's stress response. Accordingly, CRF analogs delivered to the brain should also find application in modifying the mood, learning and behavior of normal and mentally disordered individuals. Because CRF agonists elevate the levels of ACTH, $\beta$-END, $\beta$-lipotropin, other pro-opiomelanocortin gene products and corticosterone, their administration can be used to induce their effects on the brain and its periphery to thereby influence memory, mood, pain appreciation, etc., and more specifically, alertness, depression and/or anxiety, whereas CRF antagonists could be used to achieve opposite effects. Furthermore, CRF antagonists in the brain could ameliorate stress-induced conditions to which endogenous CRF might contribute, including some types of hypertension, infertility, decreased libido, impotentcy and hyperglycemia, as well as to modulate the immune system, gastrointestinal tract and adrenalcortical growth and function.

CRF analogs should also be of use for increasing blood flow to the gastrointestinal tract of mammals,

9

particularly humans and other mammals. All CRF related peptides have been shown to dialate the mesenteric vascular bed. CRF antagonists may also be of use for decreasing blood flow to the gastrointestinal tract of mammals, particularly humans. Also, oCRF inhibits gastric acid production, and CRF analogs are expected to also be effective in the treatment of gastric ulcers by reducing gastric acid production and/or inhibiting gastrointestinal functions in a mammal.

CRF analogs or the nontoxic addition salts thereof, combined with a pharmaceutically or veterinarily acceptable carrier to form a pharmaceutical composition, may be administered to mammals, including humans, either intravenously, subcutaneously, intramuscularly, percutaneously, e.g. intranasally, intracerebrospinally or orally. The peptides should be at least about 90% pure and preferably should have a purity of at least about 98%; however, lower purities are effective and may well be used with mammals other than humans. This purity means that the intended peptide constitutes the stated weight % of all like peptides and peptide fragments present. Administration to humans may be employed by a physician to lower blood pressure or to stimulate endogenous gluco-corticoid production. The required dosage will vary with the particular condition being treated, with the severity of the condition and with the duration of desired treatment.

Such peptides are often administered in the form of pharmaceutically or veterinarily acceptable nontoxic salts, such as acid addition salts or metal complexes, e.g., with zinc, iron, calcium, barium, magnesium, aluminum or the like (which are considered as addition salts for purposes of this application). Illustrative of such acid addition salts are hydrochloride, hydrobromide, sulphate, phosphate, tannate, oxalate, fumarate, gluconate, alginate, maleate, acetate, citrate, benzoate, succinate, malate, ascorbate, tartrate and the like. If the active ingredient is to be administered in tablet form, the tablet may contain a binder, such as tragacanth, corn starch or gelatin; a disintegrating agent, such as alginic acid; and a lubricant, such as magnesium stearate. If administration in liquid form is desired, sweetening and/or flavoring may be used, and intravenous administration in isotonic saline, phosphate buffer solutions or the like may be effected.

The peptides should be administered under the guidance of a physician, and pharmaceutical compositions will usually contain the peptide in conjunction with a conventional, pharmaceutically or veterinarily-acceptable carrier. Usually, the dosage will be from about 1 to about 200 micrograms of the peptide per kilogram of the body weight of the host animal. In some instances, treatment of subjects with these peptides can be carried out in lieu of the administration of ACTH or corticosteroids, in such instances a dosage as low as about 10 ng/Kg of body weight may be employed. As used herein all temperatures are ° C and all ratios are by volume. Percentages of liquid materials are also by volume.

**Claims**

1.

$$\text{H-R}_8\text{-R}_9\text{-leu-Thr-R}_{12}\text{-R}_{13}\text{-leu-leu-Arg-Glu-R}_{18}\text{-Leu-Glu-R}_{21}\text{-R}_{22}\text{-Lys-}$$

$$\text{Ala-Glu-R}_{26}\text{-R}_{27}\text{-Ala-Glu-Gln-ala-R}_{32}\text{-R}_{33}\text{-Asn-Arg-Leu-R}_{37}\text{-R}_{38}\text{-Glu-Glu-R}_{41}\text{-NH}_2$$

wherein $R_8$ is Leu or des-$R_8$; $R_9$ is Asp or des-$R_9$; $R_{12}$ is Leu or Phe; $R_{13}$ is His or Glu; $R_{18}$ is Nle or Met; $R_{21}$ is Met or Nle; $R_{22}$ is ala or Glu; $R_{26}$ is Gln or Lys; $R_{27}$ is leu or Glu; $R_{32}$ is Ala or CMA; $R_{33}$ is Leu or CML; $R_{37}$ is Leu or CML; $R_{38}$ is Leu or CML; and $R_{41}$ is Ala or CMA; or a nontoxic addition salt thereof.

2. The compound of Claim 1 wherein $R_{13}$ is His, $R_{18}$ is Nle, and $R_{26}$ is Gln.

3. The compound of Claim 2 wherein $R_8$ is Leu, $R_{12}$ is Phe, $R_{21}$ is Met, $R_{22}$ is Ala and $R_{27}$ is Leu.

4. The compound of Claim 1 wherein $R_{37}$ is Leu.

5. The compound of Claim 4 wherein $R_{27}$ is Leu, $R_{32}$ is Ala, and $R_{33}$ is Leu.

6. The compound of Claim 5 wherein $R_8$ is Leu, $R_9$ is Asp, $R_{18}$ is Met, $R_{22}$ and $R_{41}$ are Ala, $R_{26}$ is Gln, and $R_{38}$ is Leu.

7. The compound of Claim 6 wherein $R_{12}$ is Phe, $R_{13}$ is His, $R_{27}$ is Glu, $R_{32}$ is Ala, and $R_{33}$ is Leu.

8. The compound of any one of Claims 4 to 7 wherein $R_{21}$ is Met.

9. The compound of Claim 1 having either the formula:

H-Asp-Leu-Thr-Phe-His-Leu-Leu-Arg-Glu-Met-Leu-Glu-Met-Ala-Lys-Ala-Glu-Gln-Glu-Ala-Glu-Gln-Ala-Ala-Leu-Asn-Arg-Leu-Leu-Leu-Glu-Glu-Ala-NH$_2$,

or the formula:

H-Leu-Thr-Phe-His-Leu-Leu-Arg-Glu-Met-Leu-Glu-Met-Ala-Lys-Ala-Glu-Gln-Glu-Ala-Glu-Gln-Ala-Ala-Leu-Asn-Arg-Leu-Leu-Leu-Glu-Glu-Ala-NH$_2$.

10. A composition for lowering stress in mammals comprising an effective amount of a synthetic peptide or a nontoxic addition salt thereof in accordance with Claim 1 and a pharmaceutically or veterinarily acceptable liquid or solid carrier therefor.

**Claims for the following Contracting State : AT**

1. A process for the manufacture of compounds defined by the formula (I):

H-$R_8$-$R_9$-leu-Thr-$R_{12}$-$R_{13}$-leu-leu-Arg-Glu-$R_{18}$-Leu-Glu-$R_{21}$-$R_{22}$-Lys-Ala-Glu-$R_{26}$-$R_{27}$-Ala-Glu-Gln-ala-$R_{32}$-$R_{33}$-Asn-Arg-Leu-K$_{37}$-$R_{38}$-Glu-Glu-$R_{41}$-NH$_2$

wherein $R_8$ is Leu or des-$R_8$; $R_9$ is Asp or des-$R_9$; $R_{12}$ is Leu or Phe; $R_{13}$ is His or Glu; $R_{18}$ is Nle or Met; $R_{21}$ is Met or Nle; $R_{22}$ is ala or Glu; $R_{26}$ is Gln or Lys; $R_{27}$ is leu or Glu; $R_{32}$ is Ala or CMA; $R_{33}$ is Leu or CML; $R_{37}$ is Leu or CML; $R_{38}$ is Leu or CML; and $R_{41}$ is Ala or CMA; or a nontoxic addition salt thereof: comprising (a) forming a peptide having at least one protective group and having the formula II:

$X^1$-$R_8$-$R_9$($X^5$)-leu-Thr($X^2$)-$R_{12}$($X^4$)-$R_{13}$(X or $X^5$)-leu-leu-Arg($X^3$)-Glu($X^5$)-$R_{18}$-Leu-Glu($X^5$)-$R_{21}$-$R_{22}$($X^5$)-Lys($X^6$)-Ala-Glu($X^5$)-$R_{26}$($X^4$ or $X^6$)-$R_{27}$($X^5$)-Ala-Glu($X^5$)-Gln($X^4$)-ala-$R_{32}$-$R_{33}$-Asn($X^4$)-Arg($X^3$)-Leu-$R_{37}$-$R_{38}$-Glu($X^5$)-Glu($X^5$)-$R_{41}$-$X^7$

wherein: X, $X^1$, $X^2$, $X^3$, $X^4$, $X^5$ and $X^5$ are each either hydrogen or a protective group, and $X^7$ is either a protective group or an anchoring bond to resin support or NH$_2$ and (b) splitting off the protective group or groups or anchoring bond from said peptide of the formula (II) and (c) if desired, converting a resulting peptide into a nontoxic addition salt thereof.

2. The process of Claim 1 wherein, in the compound, $R_{13}$ is His, $R_{18}$ is Nle, and $R_{26}$ is Gln.

3. The process of Claim 2, wherein, in the compound, $R_8$ is Leu, $R_{12}$ is Phe, $R_{21}$ is Met, $R_{22}$ is Ala and $R_{27}$ is Leu.

4. The process of Claim 3, wherein, in the compound, $R_{37}$ is Leu.

5. The process of Claim 4, wherein, in the compound, $R_{27}$ is Leu, $R_{32}$ is Ala, and $R_{33}$ is Leu.

6. The process of Claim 5, wherein, in the compound, $R_8$ is Leu, $R_9$ is Asp, $R_{18}$ is Met, $R_{22}$ and $R_{41}$ are Ala, $R_{26}$ is Gln, and $R_{38}$ is Leu.

**7.** The process of Claim 6, wherein, in the compound, $R_{12}$ is Phe, $R_{13}$ is His, $R_{27}$ is Glu, $R_{32}$ is Ala, and $R_{33}$ is Leu.

**8.** The process of Claim 7, wherein, in the compound, $R_{21}$ is Met.

**9.** The process of Claim 8, wherein the compound has either the formula:

H-Asp-Leu-Thr-Phe-His-Leu-Leu-Arg-Glu-Met-Leu-Glu-Met-Ala-Lys-Ala-Glu-

Gln-Glu-Ala-Glu-Gln-Ala-Ala-Leu-Asn-Arg-Leu-Leu-Leu-Glu-Glu-Ala-NH$_2$,

or the formula:

H-Leu-Thr-Phe-His-Leu-Leu-Arg-Glu-Met-Leu-Glu-Met-Ala-Lys-Ala-Glu-Gln-Glu-

Ala-Glu-Gln-Ala-Ala-Leu-Asn-Arg-Leu-Leu-Leu-Glu-Glu-Ala-NH$_2$.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4 ) |
|---|---|---|---|
| Y | US-A-4 385 050 (K LEDERIS ET AL.) 24 May 1983 * claim 1 * | 1-10 | C07K7/10 A61K37/02 |
| Y | PEPTIDES, PROCEEDINGS OF THE EUROPEAN PEPTIDE SYMPOSIUM, 17TH. W. DE GRUYTER & CO, BERLIN, DE 1982, pages 597 - 603 J. RIVIER ET AL. 'solid phase synthesis of amunine (crf), sauvagine and two urotensin I' * the whole document * | 1-10 | |
| P,X | SCIENCE vol. 224, 25 May 1984, LANCASTER, PA US pages 889 - 891 J RIVIER ET AL. 'synthetic competitive antagonists of corticotropin-releasing factor: effect on ACTH secretion in the rat' * the whole document * | 1-10 | |

TECHNICAL FIELDS SEARCHED (Int. Cl.4 )

C07K
A61K

The present search report has been drawn up for all claims

| Place of search | Date of completion of the search | Examiner |
|---|---|---|
| THE HAGUE | 06 OCTOBER 1992 | p. masturzo |

EPO FORM 1503 03.82 (P0401)